# EUROPEAN PATENT SPECIFICATION

(11) **EP 0 404 877 B1**
(45) Date of publication and mention of the grant of the patent: **15.03.1995**
(21) Application number: 89910721.3
(22) Date of filing: 01.09.1989
(51) Int. Cl.: A61N 1/375, H01R 13/187, H01R 13/41

(54) **FEEDTHROUGH CONNECTOR FOR IMPLANTABLE MEDICAL DEVICE**
DURCHFÜHRUNGSSTECKER FÜR EINE IMPLANTIERBARE MEDIZINISCHE VORRICHTUNG
RACCORD DE PASSAGE D'ALIMENTATION POUR DISPOSITIF MEDICAL IMPLANTABLE

(30) Priority: 01.09.1988 US 240895
(43) Date of publication of application: 02.01.1991
(73) Proprietor: Pacesetter AB, 171 95 Solna (SE)
(72) Inventor: TRUEX, Buehl, E., Glendora, CA 91740 (US); STUTZ, William, H., Jr., Burbank, CA 91505 (US)
(74) Representative: Rees, David Christopher
(86) International application number: US8903809
(87) International publication number: WO9002581

(56) References cited:
- EP-A- 0 052 879
- EP-A- 0 251 158
- GB-A- 623 798
- GB-A- 1 598 793

## Description

The present invention relates to an electrical connector used with an implantable medical device, such as a pacemaker, for connecting an implantable electrical lead to the electrical circuits within a hermetically sealed housing of the medical device. More particularly, the present invention relates to a feedthrough/connector for use with a sealed implantable pacemaker that combines the connector function with the feedthrough function and that eliminates the need for the cast or other preformed epoxy connector that has heretofore been used on implantable pacemakers.

### Background of the Invention

Modern pacemakers monitor the activity of a heart and provide a stimulation pulse in the absence of normal heart activity. Advantageously, such devices are relatively small, light-weight and implantable. In order to sense and stimulate the heart, however, such pacemakers must be used with a pacemaker lead --an electrical conductor that carries electrical signals between the heart and the pacemaker. Advantageously, the pacemaker lead can be inserted into the heart transvenously through a relatively simple and well-known surgical procedure. Disadvantageously, one end of the lead (designated herein as the "connecting end") must be electrically and mechanically secured to the pacemaker in a way that provides for a long-term safe and secure, yet detachable, connection. Those skilled in the pacemaker art have long sought for a simple, yet reliable and safe, means for making this detachable electrical and mechanical connection between the pacemaker device and the connecting end of the pacemaker lead.

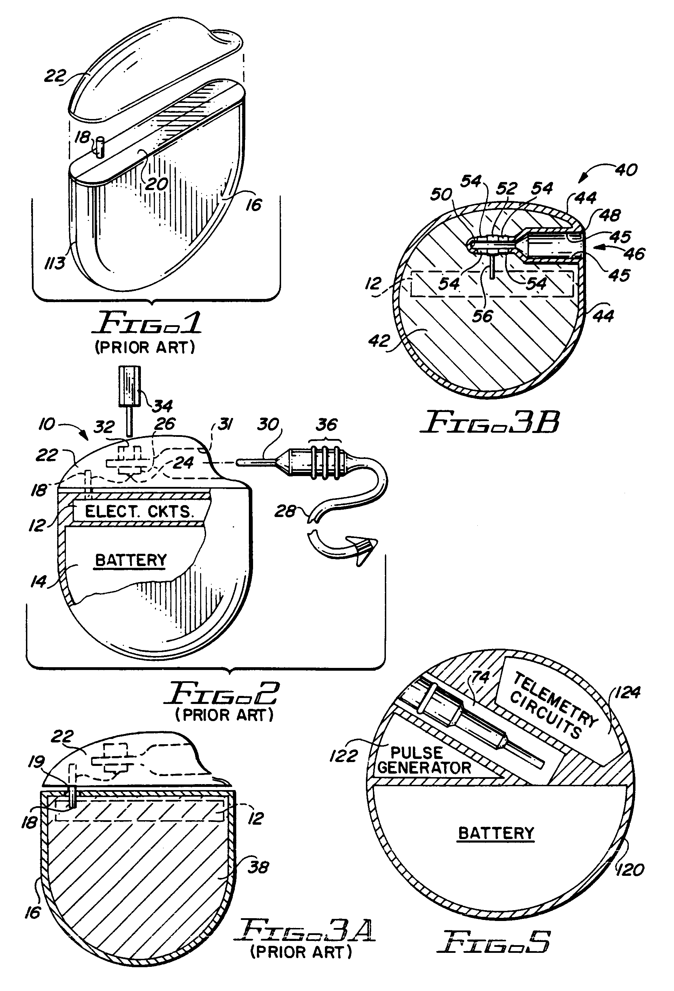

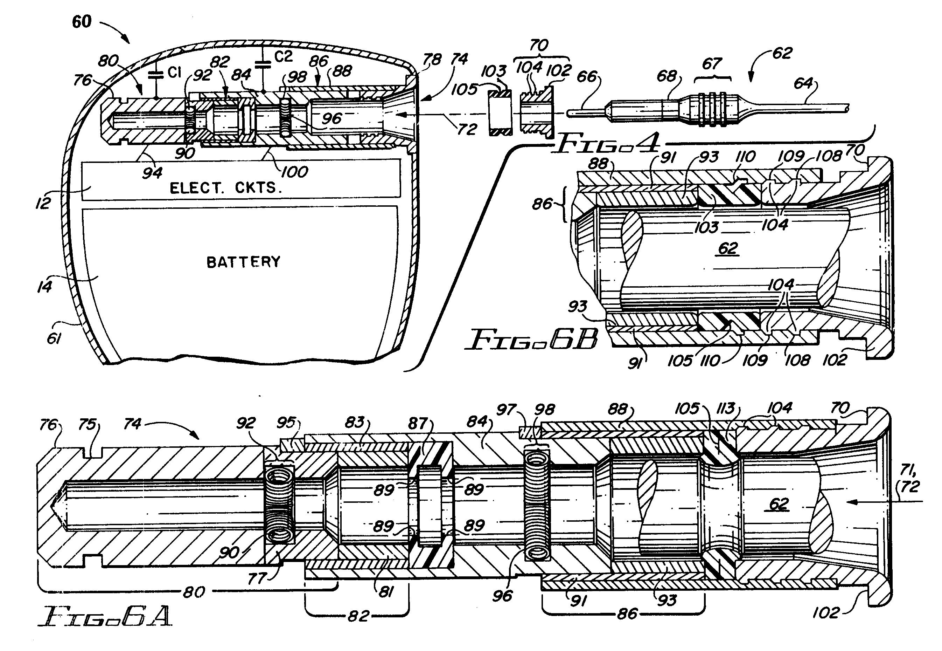

In order to appreciate the advantages of the present invention, it will help first to have a basic understanding of the manner in which the mechanical and electrical connection functions are carried out in prior art pacemakers. The main components associated with the connection function of known prior art pacemakers are shown in Figs. 1 and 2. A pacemaker 10 electrically includes a battery 14 that powers electrical circuits 12. The pacemaker electrical circuits 12 and battery 14 are mechanically housed and hermetically sealed in a suitable housing 16. Typically, this housing or case 16 is shaped to include a flat side or platform 20 to which a suitable epoxy connector 22 can be bonded. At least one feedthrough terminal 18, in electrical contact with the electrical circuits 12, passes through the case or housing 16 and protrudes out from the platform 20. This feedthrough terminal 18 is electrically isolated from the case 16. A platinum wire 24, or other suitable conductive element, connects the terminal 18 to a conductive connector block 26 that is fitted within the connector 22. A pacemaker lead 28, having a proximal electrode 30, connects to the pacemaker electrical circuits by inserting the proximal electrode 30 into a receiving channel 31 of the connector 22 until the electrode 30 is in contact with the connector block 24. A set screw 32 is then securely tightened using a torque wrench 34 to firmly hold the electrode 30 in both mechanical and electrical connection with the connector block 26. A septum (not shown) is typically placed over the set screw 32 in order to prevent body fluids from seeping through the set screw hole. Further, sealing ribs or ridges 36 on the connecting end of the pacemaker lead are designed to tightly engage the inside edges of the receiving channel 31 in order to prevent any body fluids from entering into the receiving channel 31 once the connecting end of the lead has been pushed into the connector 22.

Representative descriptions of many of the features and functions of prior art pacemaker connection systems may be found in U.S. patents: 3,683,932; 3,760,332; 4,142,532; 4,154,248; 4,182,345; and 4,316,471. While that which is described in these prior patents varies greatly relative to, for example, different types of locking mechanisms for performing the mechanical connection function, or different types of arrangements for performing the electrical feedthrough function, including the use of bipolar or multiple connector leads, all such systems include the use of a premolded or cast connector 22 that is bonded to a sealed pacemaker housing 16 in which the electrical circuits are located.

Typically, prior art connectors 22 are cast in place from epoxy to the platform or header 20 of the pacemaker, or a premolded connector is bonded to the platform 20 using a suitable sealing and bonding agent. Further, once the electrical connection is made from the terminal post 18 to the connector block 26, and the connector is attached to the housing, all remaining voids within the connector 22, not including the receiving channel 31 into which the proximal end of the lead is to be inserted, must be filled with a suitable filler material, such as a two-component epoxy or silicone rubber.

As is evident from the above description, placing a connector on a pacemaker housing is a very labor-intensive process involving many components. GB 1 598 793 discloses a case for a medical implant, said case comprising a plastics material substrate defining a closed chamber and a platinum skin fitted over the substrate. The case has a socket member defining a bore provided therein allowing electrical connection to be made to the electronic circuits inside the case. The bore of the socket member is arranged to receive an electrode lead with an appropriate plug member for electrically contacting the lead to the inside of the bore. This prior art connector arrangement comprises features corresponding to those of the preamble in the independent claims of the present application. What is needed is a simpler manner of lead attachment that provides the requisite mechanical and electrical connection functions using fewer components and less labor yet providing higher reliability. The present invention addresses these and other needs.

### Summary of the Invention

The invention is defined in the independent claims, The present invention provides a feedthrough connector for a pacemaker, or other implantable medical device, that advantageously combines the connector function with the feedthrough function and eliminates the need for the cast epoxy connector previously used on prior art pacemakers. Eliminating the external cast epoxy connector advantageously eliminates the need for septums, setscrews, and the feedthrough terminal and its associated platinum wires and connector blocks, as well as the whole time consuming casting process with its inherent propensity for cosmetic problems.

The feedthrough/connector of the present invention includes a barrel assembly having an open end and a closed end. The open end of the assembly provides an opening into which the connecting end of a pacemaker lead, or other electrical lead, can be inserted. The barrel assembly includes metal (conductive) portions separated by ceramic (nonconductive) insulating portions. An overlap region of the conductive portions, separated by the nonconductive portion, advantageously provides structural strength as well as a capacitor structure. This capacitor helps filter out unwanted electromagnetic interference (EMI) signals from passing through the connector. Spring contacts are mounted on the inside of the metal portions and are adapted to make electrical contact with the appropriate electrodes of the pacemaker or other electrical lead when the connecting end of the lead is inserted into the connector.

During assembly of the pacemaker or other device, the barrel assembly is fitted into an opening in the device housing with the open end being flush with the surface of the housing and the closed end protruding into the housing. The outer side of the metal portions are electrically connected to the appropriate electrical circuit within the housing, and the open end of the barrel assembly is welded or otherwise bonded to the device housing so that the inside of the device can be hermetically sealed. Releasable lead gripping means are included as part of the barrel assembly to detachably lock and seal the connecting end of the electrical lead in its inserted position inside of the connector.

It is a feature of the present invention to provide a feedthrough/connector system that eliminates the need for the cast epoxy type of connectors used in prior art pacemakers, and the multiplicity of problems and costs associated with the use of such cast connectors.

It is a further feature of the invention to provide a pacemaker or other implantable medical device that can be made from fewer components and that provides the requisite mechanical and electrical feedthrough functions at lower cost and higher reliability than prior art connection systems.

Still a further feature of the present invention is to provide a pacemaker that can be smaller than pacemakers of the prior art that perform an equivalent function.

Yet a further feature of the present invention is to provide a connection system for use with an implantable medical device, such as a pacemaker, that firmly yet detachably locks and seals the connecting end of an electrical lead thereto but that does not require the use of setscrews, septums, or equivalent mechanical securing and sealing devices.

A still further feature of the present invention is to provide a connection system for use with implantable medical devices that is compatible with existing electrical leads, whereby a medical device having the connection system of the present invention may replace a prior art system and still utilize an existing implantable or implanted lead that was used with the prior art system.

### Brief Description of the Drawings

The above and other features and advantages of the present invention will be more apparent from the following more particular description thereof, presented in conjunction with the following drawings, wherein:
Fig. 1 is an exploded view of a prior art pacemaker, showing the sealed pacemaker housing 16 and its cast epoxy connector top 22;
Fig. 2 is a a partial cutaway side view of a prior art pacemaker, showing the assembled relationship between the main components thereof;
Figs. 3A and 3B conceptually compare the layout and hermetically sealed area of a prior art pacemaker (Fig. 3A) and the pacemaker of the present invention (Fig. 3B);
Fig. 4 is a partial cutaway and exploded side view of a pacemaker having the feedthrough/connector of the present invention, showing the relationship between the main components thereof;
Fig. 5 is a view as in Fig. 4 of an alternative arrangement of the main components of the invention;
Figs. 6A and 6B are partial side views of the barrel assembly of the present invention showing the connector in its closed or locked position (Fig. 6A), and its open position (Fig. 6B);
Figs. 7A-7D are side sectional views (Figs. 7A, 7C, 7D) and an end view (Fig. 7B) of an alternative embodiment of the barrel assembly of the present invention wherein a collet is used as the locking means;
Fig. 8A is a side cutaway view of still an additional alternative embodiment of a portion of the barrel assembly of the present invention wherein a twist-locking bayonet-type mechanism is used to lock the lead into its inserted position within the receiving channel of the barrel assembly;
Fig. 8B is a partial sectional view taken along the line 8B-8B of Fig. 8A;
Fig. 9A is an enlarged side view of the electrical contact means used within the barrel assembly for the purpose of making multiple electrical contacts between a conductive cylindrical portion of the barrel assembly and an electrode of the pacemaker lead; and
Fig. 9B is a sectional view taken along the line 9B-9B of Fig. 9A.

### Detailed Description of the Invention

The following description presents the best contemplated mode for practicing the invention. This description is not to be taken in a limiting sense but is made merely for the purpose of describing the general principles of the invention.

The present invention is best understood with reference to the drawings, wherein like numerals are used to represent like parts or elements throughout. Where elements in one figure are similar to elements of another figure, but not the same as, such elements may be referred to using a modified reference numeral, e.g., 72' or 72'' instead of 72.

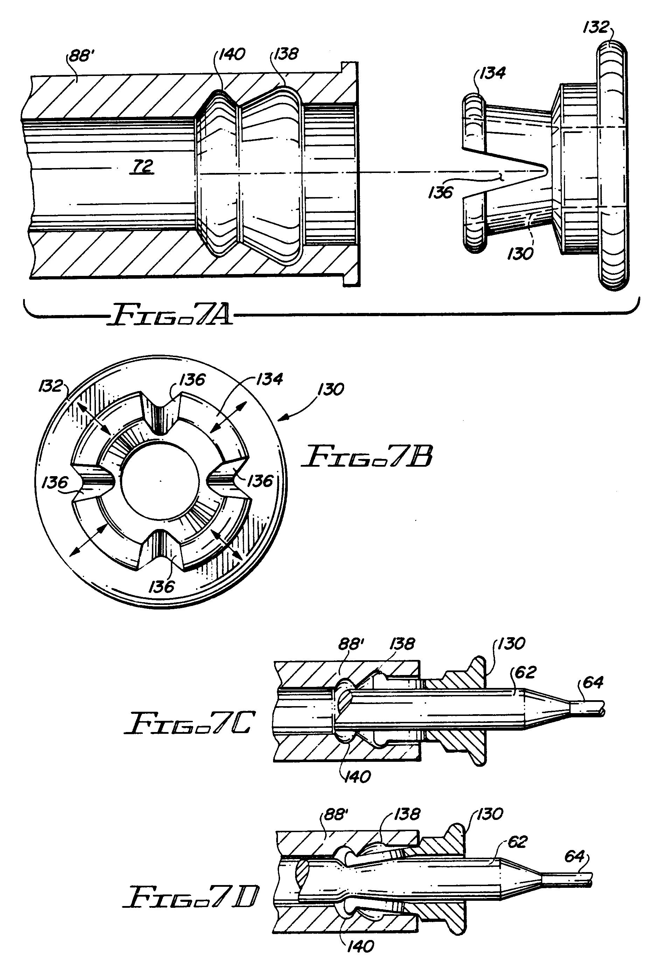

Figs. 1 and 2 have been described previously in connection with the description of the prior art in the Background portion of this application. Fig. 3A likewise depicts a prior art pacemaker device. Fig. 3A shows the relative size of the device and area therewithin that is hermetically sealed. The sealed area is the shaded area 38. As has been indicated, the electrical circuits 12 of the pacer are housed in the sealed area 38. In order to make electrical contact with these circuits 12, at least one feedthrough terminal 18 must pass through the case 16. Some type of insulating material 19 must be used with the feedthrough terminal 18 in order to electrically insulate this terminal from the case 16. (A portion of the case 16 typically functions as a return electrode for unipolar pacing.)

Fig. 3A should be compared to Fig. 3B, where there is shown a simplified side sectional view of a pacemaker 40 incorporating the present invention. In Fig. 3B, that portion of the pacemaker that is hermetically sealed is the shaded area 42. This area is enclosed by the case 44 of the pacemaker. As with the prior art device, this sealed area 42 includes the electrical circuits 12 of the pacer. Unlike the prior art device, a receiving channel 46 protrudes inwardly into the pacer. This channel may conceptually be thought of as an indented channel for it includes an open end 48 flush with the surface of the pacer housing 44 and a closed end 50 within the pacer housing 44, thereby forming, as it were, a long narrow indent within the pacer housing 44. (As will be described more fully below, the receiving channel 46 is not formed by indenting the pacer housing 44. Nonetheless, for purposes of illustrating those areas of the pacer that are sealed from those areas that are not, it may be helpful to conceptualize the receiving channel as an indented channel.) The inside walls 45 of the receiving channel 46 are not included within the sealed areas of the pacemaker 40 for they are open to the outside environment of the pacer through the open end 48. In contrast, the reverse side of the inside walls 45 of the receiving channel 46 (referred to hereafter as the "backside" or "outside" walls of the receiving channel 46) are exposed to the sealed inner portions of the pacemaker.

Still referring to Fig. 3B, a portion 52 of the walls of the receiving channel 46 are made from a conductive material, such as a short 316L stainless tubular section. This conductive portion is insulated from the pacer walls 44 by insulating (nonconductive) portions 54 of the walls of the receiving channel 46 which are adjacent the conductive portion. Typically, as is explained more fully below, these nonconductive portions 54 may be made from short ceramic tubular sections that are hermetically bonded to the conductive portion 52 and the walls of the channel 45. However, any suitable nonconductive material, such as an epoxy or polymer substance, could be used to perform this insulating function providing that a suitable hermetic bond is made thereto.

The backside or outside of the conductive portion 52 is electrically connected to the pacemaker circuits 12 by means of a suitable electrical conductor 56. Advantageously, because the conductor 56 is only included within the sealed portion 42 of the pacer, it can be made from any suitable electrically conductive material, not just those types of conductors (such as titanium) that are compatible with exposure to body fluids. However, the conductor 56 should be made from a material that is compatible with the type of material used for the conductive portion 52 of the receiving channel 46 in order to prevent any galvanic or other adverse reactions between dissimilar metals in electrical contact with each other.

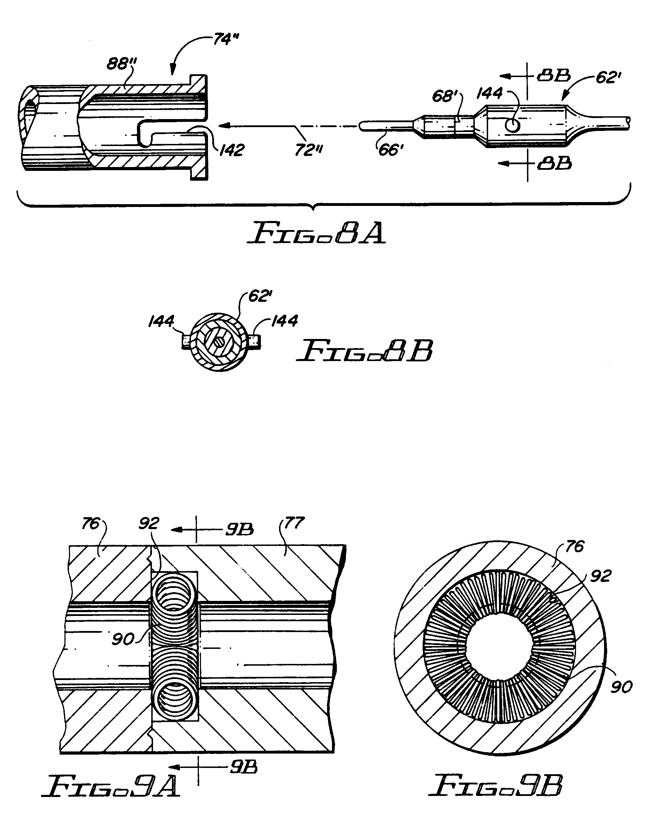

Referring now to Figs. 4, 6A and 6B, a more detailed cutaway side and sectional views of a pacemaker 60 embodying the present invention is shown. Included in Fig. 4 is a side view of the connecting end 62 of a pacing lead 64 adapted to be inserted into the connector of the present invention, as well as a sectional side view of a lead lock button 70 and sealing/gripping element 103. (For clarity, the lead lock button is shown in Fig. 4 in an exploded position from its normal position within the open end of the receiving channel 72 of the connector.) The lead lock button 70 and sealing/gripping element 103 lock and seal the connecting end 62 of the lead 64 into a receiving channel 72 of the feedthrough connector. It is noted that the lead 64 shown in Fig. 4 is a bipolar lead, having a connecting end tip electrode 66 and a connecting ring electrode 68, both of which electrodes must make electrical contact with the electrical circuits 12 of the pacemaker 60. It is further noted that in recent years there has been an effort underway in the pacemaker industry to voluntarily standardize pacemaker connectors, at least insofar as the size, spacing, and shape of the connecting end of the pacing lead is concerned, thereby allowing an implanted lead in a patient to be used with any manufacturer's pacemaker. That which is shown as the proximal end 62 in Fig. 4 is intended to depict that which is known as a voluntary standard VS-1A lead. The VS-1A lead is a bipolar lead having specific dimensions. The specifications associated with the VS-1A connecting end may be found in public domain documents, such as IEC SC62D/WG6 and ISOTC150/SC2/WG2 (July 1987); and Voluntary Standard VS-1 (June 1986). Other standardized connecting ends include the VS-1 lead and VS-1B lead. One of the advantageous features of the feedthrough connector described herein is that it is compatible for use with all of these industry standard pacing lead connectors. Another desired feature of the invention allows the connecting end 62 of the lead to be slidably inserted into the connector without having to rotate either the lead or the case relative to each other, which rotation (typically involving a plurality of turns, e.g., a rotation of more than 360 degrees) was required in some very early prior art pacemakers. See, e.g., U.S. Patent No. 3,871,382.

Figs. 4 and 6A illustrate the manner in which the feedthrough connector of the present invention is fabricated. A barrel assembly 74 is constructed which, when assembled, defines the receiving channel 72. The barrel assembly is shown in its inserted position within a pacemaker housing in Fig. 4. The barrel assembly is shown by itself in an enlarged view in Fig. 6A in order to better illustrate some of the details associated therewith. The barrel assembly 74 includes tubular sections of conductive and nonconductive materials that are hermetically joined together. A blind hole end piece 76 closes one end of the assembly, and the opposite end 78 is welded to the device housing 61. A first conductive section 80 of the barrel assembly 74 includes a spring contact 90 within a groove 92. As shown best in Fig. 6A, this first conductive section 80 comprises the blind hole end piece 76 welded to an adjoining conductive section 77. A counter bore is machined into one end of the section 77. During assembly, the spring contact 90 is placed into the bore 79 prior to welding the end piece 76 to the section 77. Once this weld is completed, the groove 92 is formed (by the bore and the end of the end piece 76) , which groove maintains the spring contact 90 in its desired position. Both the end piece 76 and the section 77 are preferably made from 316L stainless steel.

A second conductive section 84 of the barrel assembly 74 is bonded to the spring contact end of the first conductive section 80 by means of a first nonconductive section 82. In the preferred embodiment, the nonconductive section 82 comprises a rigid portion 81 and a seal portion 83. Preferably the rigid portion 81 is made from a hard relatively nonmelting ceramic bead or ring, and the seal portion 83 is made from Kryoflex™, a form of meltable ceramic available from Kyle Technology, of Roseburg, Oregon. The seal portion 83, upon being subjected to sufficient heat for a prescribed period of time, melts and fuses with the adjacent conductive sections 77 and 84 as well as the rigid ceramic section 81 in order to form a suitable hermetic bond and seal.

A second spring contact 96 is placed within a groove 98 located around the inside of one end of the second conductive section 84. A silicone seal 87 may optionally be placed within a suitable bore at the other end of the second conductive section 84. (Besides the seal 87, the first nonconductive section 82 and a portion of the conductive piece 77 are also fitted within this same bore.) The silicone seal 87, which includes a plurality of sealing ribs 89, tightly encircles the connecting end 62 of the lead 64 when the lead is inserted into the receiving channel 72, thereby preventing body fluids from coming in contact with the first conductive section 80. As will be explained more fully hereinafter, the seal 87 provides only a passive and secondary seal for the connector. An active and primary seal is provided by the lead lock button 70 and its associated sealing/gripping element 103. For this reason, there may be some applications where the seal 87 may not be needed.

A third conductive section 88 of the barrel assembly 74 is similarly bonded to the spring contact end of the second conductive section 84 by means of a second nonconductive section 86. This second nonconductive section 86 is similar to the first nonconductive section 82 in that it comprises a rigid portion 91 and a seal portion 93, with the seal portion 93 melting and fusing with the adjacent conductive sections 84 and 88 and the rigid ceramic 93 upon being subjected to adequate heat for a prescribed time period.

As also seen in Fig. 6A, a positioning groove 75 is placed around the backside of the first conductive section 80 of the barrel assembly 74. This positioning groove 75 is used during manufacture of the pacer in order to correctly position and support the assembly within the pacemaker or other device. Further, during assembly of the barrel 74, ceramic spacer rings 95 and 97 may be optionally used around the backside of conductive sections 80 and 84, respectively, in order to assure that these conductive sections are inserted into the adjacent conductive sections the appropriate depth prior to firing the ceramic bond and seal.

In the preferred embodiment, as shown best in Fig. 6A, the conductive section 80 overlaps the conductive section 84 with the nonconductive section 82 being inserted therebetween. Advantageously, this overlap allows the mechanical strength of the conductive sections to overlap and protect the relatively weaker nonconductive sections, thus assuring that there are not weak sections of the barrel assembly that could easily break. A similar overlap occurs between the conductive section 84 and the conductive section 88, with the nonconductive section 86 being inserted therebetween.

Further, this overlap advantageously provides the structure of a capacitor. That is, a first conductive plate (e.g., one end of the conductive section 88) is uniformly spaced apart from a second conductive plate (e.g., one end of the conductive section 84) by a dielectric nonconductive material (e.g., the nonconductive section 86). These built-in capacitors (there are two such capacitors) advantageously provide an electrical filter for filtering out unwanted signals, such as EMI, from the signals present on the first and second conductive portions, 80 and 84, of the barrel assembly. Further, these built-in capacitors may be supplemented, as required, with external capacitors, such as capacitors C1 and C2 (Fig. 4), placed within the sealed portion of the pacemaker housing.

The conductive sections 80 and 84 are preferably made from 316L stainless steel. Conductive section 88 is made from titanium to facilitate welding it to the housing 61. As has been indicated, nonconductive sections 82 and 86 are preferably made from a ceramic material, including a rigid portion and a seal or meltable portion. Other suitable conductive and nonconductive materials could, of course, be used.

As already mentioned, included within the first conductive section 80 is a spring contact 90 that is fitted within a groove 92 formed within an inside wall of the section 80. This spring contact is preferably a canted coil spring, commonly referred to as a garter spring. It makes multiple electrical contacts with the conductive sections 76 and 77 around the periphery of the groove 92. Further, when the connecting end 62 of the lead 64 is inserted into the receiving channel 72, this spring 90 makes multiple electrical contacts with the connecting tip electrode 66. A suitable electrical conductor 94, in electrical contact with the back side of the conductive section 80 is also in electrical contact with the electrical circuits 12 (Fig. 4). Thus, by means of the garter spring 90, which is in electrical contact with the inside of the conductive section 80, and the conductor 94, which is in electrical contact with both the back side of the conductive section 80 and the electrical circuits 12, the connecting tip electrode 66 is placed in electrical contact with the electrical circuits 12 which are hermetically sealed in the pacemaker housing.

In a similar fashion, the second spring contact 96 is placed within a groove 98 around the inside of the second conductive section 84. The backside of this second conductive section 84 is electrically connected to the pacemaker circuits 12 by means of a second conductor 100. This second spring contact 96, which is also a canted coil spring, or garter spring, makes multiple electrical contact with the connecting ring electrode 68 of the pacing lead 64 when such lead is inserted into the receiving channel 72 of the connector.

The garter or canted coil springs 90 and 96 comprise helically wound spring elements configured in a circle, thus forming doughnut-shaped elements. As indicated above, these springs advantageously provide multiple electrical contacts around the entire periphery of the elements with which they come in contact, as shown best in Figs. 9A and 9B. Such garter springs are commercially available from, for example, Bal-Seal Corporation, of Santa Ana, California.

Still referring to Figs. 4, 6A, and 6B, a lead lock button 70 will now be described. This button provides a mechanism for locking the connecting end 62 of the lead 64 into its desired position within the receiving channel 72 of the feedthrough connector. As is evident from the figures, the lead lock button 70 is a relative short annular element having an opening 71 through the center thereof. This opening has a diameter sufficiently large to allow the connecting end 62 of the pacemaker lead 64 to be snugly passed therethrough. The button 70 further has a circumferential lip 102 at one end thereof (used to facilitate gripping the button as it is slid between its open and locked positions in the receiving channel 72) and a plurality of small detent bumps 104 near the other end, used to hold the button in either of its two positions. A short section of tubular or annular resilient material 103, such as silicon rubber, is held captive within the receiving channel 72 when the lead lock button is inserted. This annular resilient material 103 functions much like a collar having an adjustable inner diameter. When the lead lock button is in one position, the diameter of the collar allows the electrical lead to pass therethrough. When the lead lock button is in its other position, explained below, the collar assumes a much smaller diameter that pinches the lead, thereby preventing the lead from any axial movement.

The resilient material 103 has a circumferential groove 105 on the outside diameter thereof to allow it to bulge or bend inwardly upon axial compression, as explained below. The material from which the button is made, in conjunction with the thickness of the walls of the button 70, and the plurality of detent bumps 104 (which engage circumferential grooves around the inside of the third conductive section 88) allow the button to snap into an open or locked position in much the same manner as the cap of a felt-tip pen (which includes one or more circumferential grooves) snaps on to the pen body (which includes a plurality of detent bumps).

Included around the inside circumference of the third section 88 of the barrel assembly 74 are spaced-apart grooves 108, 109 and 110. These grooves are adapted to receive the detent bumps 104 of the lead locking button 70. Once the lead locking button is slidably inserted into the receiving channel 72, the combination of detent bumps and grooves allow for two positions of the button 70: (1) a captive open position (Fig. 6B) that allows the lead 62 to freely pass into or out of the receiving channel 72; and (2) a closed position (Fig. 6A) wherein the lead 62 is locked into the receiving channel. In the open position (Fig. 6B), the detent bumps 104 engage grooves 108 and 109, thus holding the locking button captive within the connectors and the resilient material 103 is held captive between the end of the nonconductive sections 91, 93 and the end of the lead lock button 70. In this open position, the material 103 is not substantially axially compressed, and the lead 62 can pass therethrough. In the closed or locked position (Fig. 6A), the detent bumps 104 engage grooves 109 and 110, and the resilient material 103 is subjected to a significant axial compression, which compression causes it to fold or bend at the groove 105 and bulge radially inward into the receiving channel 72. This action causes the resilient material 103 to firmly grip and compress the lead 62 around its circumference, much like a shrinking collar. Advantageously, this gripping action further provides an active (under pressure) seal that totally and completely blocks the entry of any body fluids into the receiving channel 72.

In the preferred embodiment, the resilient tubular material 103 is a noncompressible elastomer, such as silicone or urethane. The gripping action on the lead locking button 70 places the elastomer of the lead lock mechanism in firm contact with the silicon rubber of the pacing lead. As has been indicated, this action not only provides a very effective (tight) and active (under pressure) seal, but it also provides a very firm grip or lock because of the high coefficient of friction of these materials. The lead locking button 70 is preferably made from a nonconductive biocompatible thermoplastic resin, such as polysurfone or nylon.

It is also noted that sealing ridges 67 placed around the circumference of the connecting end 62 of the lead 64 in accordance with the VS-1A standard (or other standards) are tightly received within the opening 71 of the lead lock button 70 and/or the receiving channel 72. These ridges provide a secondary or back-up passive seal that further prevents any body fluids from entering the channel 72, just as do the silicone seal ridges 89 previously described.

Referring back to Fig. 4, it is noted that the case 61 of the pacemaker 60 is typically assembled in halves, commonly known as clamshells, with the two halves being welded together around their periphery once all of the electrical components have been placed therein. (The welding seam for a prior art pacemaker, for example, is shown as 113 in Fig. 1) In accordance with the present invention, such assembly techniques can continue to be used. That is, once the barrel assembly 74 has been assembled, it is positioned within a suitable opening within that half of the pacemaker 60 containing the other electrical components (circuits 12 and battery 14). After the appropriate electrical connections are made between the backside of the conductive elements 80 and 84 and the electrical circuits 12, and after the filter capacitors C1 and C2 are installed (if such are used), and after other conventional assembly steps are completed, the other half of the pacemaker case is welded or otherwise hermetically bonded to the half of the pacemaker case containing all of the pacemaker elements, and the open end 72 of the barrel assembly 74 is welded or otherwise bonded to both pacemaker case halves, in order to hermetically seal the entire pacemaker case. In a preferred assembly process, the hermeticity of the barrel assembly provides a measured leak rate of no greater than 2 x 10E-9 atm-cc/sec of air, when tested in accordance with MIL-STD-883, method 1014, condition A. The hermeticity of the pacer assembly may be somewhat less than this (i.e., a slightly higher measured rate of leakage) due to the aging of the barrel assembly, which aging may degrade the hermeticity level somewhat. For example, a hermeticity level for the pacer assembly of 2 x 10⁻⁹ Nm/s (2 x 10E-8 atm-cc/sec) would be acceptable.

Referring next to Figs. 7A-7D, an alternative embodiment of the lead locking means of the present invention is illustrated. As shown best in Figs. 7A and 7B, this embodiment utilizes a collet 130 that is adapted to be slidably inserted into the receiving channel 72' of the feedthrough connector. The collet 130 includes a circumferential lip 132 (used to help push the collet into and pull the collet out of the receiving channel 72') and an engaging detent rib 134. Four slits 136, uniformly spaced around the periphery of the body of the collet, allow the detent rib 134 to be radially compressed. However, the material from which the collet is made, e.g. an engineering biocompatible thermoplastic resin (such as polysurfone or nylon), provides a spring bias that tends to force the rib 134 in an outwardly radial direction. Receiving grooves 138 and 140 in the third section 88' of the barrel assembly are designed to receive the rib 134 as the collet 130 is pushed into the receiving channel. The diameter of the first groove 138 is larger than the diameter of the second groove 140. Hence, when the detent rib 134 is received in the first groove 138, the collet has not been compressed to the point where the lead 64 cannot pass therethrough. Thus, with the detent rib 134 residing in the first groove 138, the collet is in its "open" position, and the lead 64 can be inserted into the receiving channel 72' of the connector. This open position is illustrated in Fig. 7C. Note that the collet 130 is held captive within the connector in this open position, thereby preventing the collet from becoming misplaced. However, when the collet 130 is further slid into the receiving channel 72', such that the detent rib 134 engages the second groove 140, the collet is compressed to the point where it squeezes the body of the connecting end 62 of the lead 64 and firmly locks the lead into its inserted position. Thus, with the detent rib 134 residing in the second groove 140, the collet is in its "locked" position and the lead can not be removed from the connector. This locked position is shown in Fig. 7D. The lead is released by using a suitable tool to engage the circumferential lip or rim 132 so that the collet can be pulled or slid from its "locked" position to its "open" position.

Referring next to Figs. 8A and 8B, another alternative embodiment of the lead locking means of the present invention is shown. In accordance with this embodiment, a barrel assembly 74'' includes a plurality of keyed channels 142 along the inside of a third section 88'' of the barrel assembly. These keyed channels run longitudinally for a short distance and then make a right angle and run circumferentially. Protruding pins 144, or equivalent engaging elements, are placed into the proximal end 62' of the pacing lead. These pins 144 are received within the keyed channels 142 when the lead is inserted into the receiving channel 72'' of the connector. Once the pins have traversed the longitudinal length of these channels 142, the lead is rotated relative to the receiving channel 72'', thereby placing the pins 144 at the extreme end of the keyed channels 144. With the pins in this position, the lead cannot be longitudinally removed from the receiving channel 72''. Hence, the lead is locked into the connector. Removal of the lead is accomplished by twisting the lead relative to the connector in the other direction until the pins are aligned with the longitudinal portion of the channel 142, at which time the lead can be pulled back out of the connector. This type of lead locking mechanism is commonly known as a bayonet-type engagement. Typically, less than 1/8 of a turn is required to lock or unlock the lead. This is felt to provide a significant improvement over prior art devices where the lead must be threadably inserted into a recess within the pacemaker, typically involving many turns, and always involving relative rotation between the case and lead in excess of 360 degrees.

Fig. 5 illustrates an alternative manner in which the barrel assembly 74, of the type previously described in connection with Figs. 4 and 6A, could be used with a circular pacemaker case 120. In such an arrangement, the location of the pacemaker circuits within the housing is altered slightly in order to better utilize the available space within the device. For example, Fig. 5 depicts the pacemaker electrical circuits as being divided into two portions: the pulse generator circuits 122 and the telemetry circuits 124. Those skilled in the pacemaker art could readily divide the pacemaker circuits into these, or other, groups for the purpose of optimally utilizing the available space within the pacemaker, thereby allowing the pacemaker to be as small as possible.

## Claims

1. An implantable medical device comprising:
a hermetically sealed housing (61);
an electrical circuit (12) within said housing;
a connector within said housing comprising:
a tubular barrel assembly (74) having an open end (78) and a closed end (76), and a barrel axis passing through the open end (78) and closed end (76), said open end (78) being attached to an exposed surface of said housing (61), said closed end (76) being internal to said housing (61), the tubular barrel assembly (74) creating a tubular channel (72) that protrudes into the sealed housing (61), but does not break the seal of said housing (61), the inside of said tubular channel (72) being open to the outside of said sealed housing (61) through said open end (78),
means for making electrical contact between said tubular channel (72) and said electrical circuit (12), and
locking means (70) for detachably locking and gripping an electrical lead (64) axially inserted into the open end (78) of said tubular channel (72), said locking means being actuable with less than 360 degrees of relative rotation between said lead and said tubular barrel assembly, **characterized in** that
said tubular channel (72) further comprises a plurality of conductive cylindrical portions (80, 84) coaxial with the barrel axis, the dimension of the diameter of successive cylindrical portions (80, 84) progressively decreasing from the open end (78) to the closed end (76);
said electrical contact being made between selected ones of said cylindrical portions (80, 84) and said electrical circuit (12); and said electrical lead (64) being in electrical contact with said selected ones of said cylindrical portions (80, 84) of said tubular channel (72) (Fig. 4, 6);
wherein successive ones of the plurality of conductive cylindrical portions (80, 84) are separated by an insulating non-conductive cylindrical portion (82, 86);
and wherein successive conductive cylindrical portions (80, 84) of said barrel assembly (74) are spaced apart and overlap at their respective adjacent ends, thereby defining a region of overlap therebetween, the insulating nonconductive cylindrical portions (82, 86) being disposed throughout the region of overlap between successive conductive cylindrical portions (80, 84), said ends of said conductive cylindrical portions (80, 84) being thereby held in a spaced-apart nontouching and electrically isolated relationship by said insulating nonconductive cylindrical portions (82, 86).

2. The implantable medical device of claim 1 wherein said hermetically sealed housing (61) shall not exhibit a leak rate of greater than 2 x 10⁻⁹ Nm/s (2 x 10E-8 atm-cc/sec).

3. The implantable medical device of claim 1 wherein said hermetically sealed housing (61) comprises a pacemaker (60), and said electrical lead (64) comprises a pacemaker lead (64) adapted to connect the pacemaker (60) to a desired tissue location.

4. The implantable medical device of claim 1 wherein said locking means (70) is actuable with substantially no relative rotation between said electrical lead (64) and said tubular barrel assembly (74).

5. The implantable medical device of claim 1 wherein said means for making electrical contact comprises:
a canted coil spring (90, 96) positioned on the inside of said selected cylindrical portions 80, 84) so as to make electrical contact therewith in a multiplicity of locations; and
wire means (94, 100) for making electrical contact with the outside of said selected cylindrical portions (80, 84) and said electrical circuit (12).

6. The implantable medical device as defined in any preceding claim, wherein said locking means comprises first (109, 110) and second (108, 109) spaced-apart
engaging means on the inside of said tubular channel;
a locking ring (70) having a plurality of detent bumps (104), said locking ring (70) having an inside diameter that allows said electrical lead (64) to fit therethrough, and an outside diameter that allows said ring (70) to be slidably inserted into the open end (78) of said tubular channel (72), at least one detent bump (104) of the locking ring (70) being received by either said first (109, 110) or second (108, 109) engaging means depending upon the depth to which the locking ring (70) is inserted into the tubular channel (72);
gripping means for gripping said electrical lead whenever said at least one detent bump (104) is received by said first engaging means (109, 110), and for releasing said electrical lead whenever said at least one detent bump (104) is received by said second engaging means (108, 109).

7. The implantable medical device of claim 6 wherein said first (109, 110) and second (108, 109) engaging means comprise first (109, 110) and second (108, 109) circumferential grooves on the inside of said tubular channel (72) near the open end (78) thereof; and wherein said at least one detent bump (104) of said locking ring (70) snaps into either said first (109, 110) or second (108, 109) groove depending upon the depth to which the locking ring (70) is pushed into the tubular channel (72).

8. The implantable medical device of claim 7 wherein the tubular channel (72) of the barrel assembly (74) further includes stop means positioned near said first (109, 110) and second (108, 109) circumferential grooves, and wherein said gripping means is held captive between said stop means and said locking ring (70), said gripping means comprising an annular section (103) of resilient material, having an inside diameter that allows said electrical lead (64) to pass therethrough when said resilient material is not subjected to axial compression, and further wherein said resilient material is placed under axial compression when said at least one detent bump (104) is received within said first groove (109, 110), said axial compression causing the resilient material to bulge inwardly into the tubular channel (72), thereby reducing the inside diameter of said annular resilient section (103), sufficiently to firmly grip the electrical lead (64) thereby preventing axial movement thereof.

9. The implantable medical device of claim 7 wherein the diameter of the first groove (138) is larger than the diameter of the second groove (140), and wherein the locking ring comprises a radially compressible collet (130), said collet having a first inside diameter that allows said electrical lead (64) to slidably pass therethrough when the shoulder of the collet (130) is received within said first groove (138), and wherein said collet (130) has a second inside diameter that lockably grips said electrical lead (64) when the shoulder of the collet is received within the second groove (140) (Fig. 7).

10. The implantable medical device of claim 1 wherein said locking means comprises engaging means on the inside of said tubular channel (72'') that mate with matching engaging means (144) on said electrical lead (62') whenever a proximal end of said electrical lead (62') is inserted into the open end of said tubular channel (72'') (Fig. 8).

11. The implantable medical device of claim 10 wherein said engaging means of said tubular channel (72'') and said electrical lead (62') become lockably engaged so as to prevent removal of said electrical lead (62') from said tubular channel (72'') whenever said electrical lead (62') is rotated relative to said tubular channel (72'') a fraction of a turn (Fig. 8).

## Patentansprüche

1. Eine implantierbare medizinische Vorrichtung mit:
einem hermetisch abgedichteten Gehäuse (61);
einer elektrischen Schaltung (12) in dem Gehäuse;
einem Anschluß in dem Gehäuse mit:
einem rohrförmigen Zylinderaufbau (74) mit einem offenen Ende (78) und einem geschlossenen Ende (76) und einer Zylinderachse, die durch das offene und das geschlossenen Ende hindurchgeht, wobei das offene Ende (78) mit einer freien Oberfläche des Gehäuses (61) verbunden ist und das geschlossene Ende (76) innerhalb des Gehäuses (61) liegt, wobei der rohrförmige Zylinderaufbau (74) einen rohrförmigen Kanal (72) erzeugt, der in das abgedichtete Gehäuse (61) hineinragt, aber die Dichtung des Gehäuses (61) nicht unterbricht, und wobei das Innere des rohrförmigen Kanals (72) gegen die Außenseite des abgedichteten Gehäuses (61) über das offene Ende (78) geöffnet ist,
Mitteln zum Herstellen eines elektrischen Kontaktes zwischen dem rohrförmigen Kanal (72) und der elektrischen Schaltung (12) und
einer Verriegelungsvorrichtung (70) zum trennbaren Arretieren und Einspannen einer elektrischen Elektrodenvorrichtung (64), die axial in das offenen Ende (78) des rohrförmigen Kanals (72) eingeführt ist,
wobei die Verriegelungsvorrichtung mit weniger als 360 Grad relativer Rotation zwischen der Elektrodenvorrichtung und dem rohrförmigen Zylinderaufbau betätigbar ist, **dadurch gekennzeichnet**, daß der rohrförmige Kanal (72) weiterhin eine Mehrzahl leitender zylindrischer Abschnitte (80, 84) koaxial zur Zylinderachse aufweist, wobei die Dimension des Durchmessers aufeinanderfolgender Abschnitte (80, 84) progressiv vom offenen Ende (78) zum geschlossenen Ende (76) abnimmt;
der elektrische Kontakt zwischen ausgewählten zylindrischen Abschnitten (80, 84) und der elektrischen Schaltung (12) hergestellt wird; und die Elektrodenvorrichtung (64) in elektrischem Kontakt mit den ausgewählten zylindrischen Abschnitten (80, 84) des rohrförmigen Kanals (72) steht (Fig.4,6);
wobei aufeinanderfolgende der Mehrzahl der leitenden zylindrischen Abschnitte (80, 84) durch einen isolierenden, nichtleitenden zylindrischen Abschnitt (82, 86) separiert sind;
und wobei aufeinanderfolgende leitende zylindrische Abschnitte (80, 84) des Zylinderaufbaus (74) voneinander getrennt sind und sich an ihren benachbarten Enden überlappen, wodurch sie Überlappungsbereiche zwischen sich definieren, und die isolierenden, nichtleitenden zylindrischen Abschnitte (82, 86) im Überlappungsbereich zwischen benachbarten leitenden zylindrischen Abschnitten (80, 84) angeordnet sind, wodurch sich die Enden der leitenden zylidrischen Abschnitte (80, 84) durch die isolierenden, nichtleitenden zylindrischen Abschnitte (82, 86) in einem getrennten, sich nicht berührenden und elektrisch isoliertem Verhältnis befinden.

2. Implantierbare medizinische Vorrichtung nach Anspruch 1, bei der das hermetisch abgedichtete Gehäuse (61) keine Leckrate größer 2x 10⁻⁹ Nm/s (2x 10E-8 atm-cc/sec) aufweist.

3. Implantierbare medizinische Vorrichtung nach Anspruch 1, bei der das hermetisch abgedichtete Gehäuse (61) einen Herzschrittmacher (60) und die elektrische Elektrodenvorrichtung (64) eine Herzschrittmacherelektrodenvorrichtung (64) aufweist, die angepaßt ist, um den Herzschrittmacher (60) mit einer gewünschten Gewebestelle zu verbinden.

4. Implantierbare medizinische Vorrichtung nach Anspruch 1, bei der die Verriegelungsvorrichtung (70) im wesentlichen ohne relative Rotation zwischen der elektrischen Elektrodenvorrichtung (64) und dem rohrförmigen Zylinderaufbau (74) betätigbar ist.

5. Implantierbare medizinische Vorrichtung nach Anspruch 1, bei der die Mittel zum Herstellen eines elektrischen Kontaktes aufweisen:
eine abgeschrägte Spiralfeder (90, 96), die auf der Innenseite der ausgewählten zylindrischen Abschnitte (80, 84) so angeordnet ist, daß sie damit an einer Vielzahl von Stellen elektrischen Kontakt herstellt; und
Drähten (94, 100) zur Herstellung eines elektrischen Kontaktes zwischen der Außenseite der ausgewählten zylindrischen Abschnitte (80, 84) und der elektrischen Schaltung (12).

6. Implantierbare medizinische Vorrichtung nach einem der vorhergehenden Ansprüche, bei der die
Verriegelungsvorrichtung erste (109, 110) und zweite (108, 109) örtlich getrennte
Verbindungsmittel auf der Innenseite des rohrförmigen Kanals;
einen Sperring (70) mit einer Mehrzahl von Arretiererhebungen (104), wobei der Sperring (70) einen Innendurchmesser hat, der es erlaubt, die elektrische Elektrodenvorrichtung (64) hindurchzuführen, und einen Außendurchmesser, der es erlaubt, daß der Ring (70) gleitend in das offenen Ende (78) des rohrförmigen Kanals (72) eingeführt wird, mindestens eine Arretiererhebung (104) des Sperringes (70) in Abhängigkeit von der Tiefe, bis zu der der Sperring (70) in den rohrförmigen Kanal (72) eingeführt wird, von entweder dem ersten (109, 110) oder dem zweiten (108, 109) Verbindungsmittel empfangen wird;
Einspannmittel zum Einspannen der elektrischen Elektrodenvorrichtung, wann immer wenigstens die eine Arretiererhebung (104) von den ersten Verbindungsmitteln (109, 110) empfangen wird, und zur Freigabe der elektrischen Elektrodenvorrichtung, wann immer wenigstens die eine Arretiererhebung (104) von dem zweiten Verbindungsmittel (108, 109) empfangen wird, aufweisen.

7. Implantierbare medizinische Vorrichtung nach Anspruch 6, bei der die ersten (109, 110) und die zweiten (108, 109) Verbindungsmittel kreisförmige Vertiefungen auf der Innenseite des rohrförmigen Kanals (72) nahe dem offenen Ende (78) aufweisen; und bei der wenigstens eine der Arretiererhebungen (104) der Sperrvorrichtung (70) entweder in die erste (109, 110) oder die zweite (108, 109) Vertiefung einrastet, je nachdem, bis zu welcher Tiefe der Sperring (70) in den rohrförmigen Kanal (72) hineingedrückt wird.

8. Implantierbare medizinische Vorrichtung nach Anspruch 7, bei der der rohrförmige Kanal (72) des Zylinderaufbaus (74) weiterhin in der Nähe der ersten (109, 110) und der zweiten (108, 109) kreisförmigen Vertiefungen angeordnete Stoppmittel aufweist und bei der die Einspannmittel zwischen den Stoppmitteln und dem Sperring (70) festgehalten werden, wobei die Einspannmittel einen ringförmigen Abschnitt (103) aus elastischem Material aufweisen, der einen Innendurchmesser hat, der es erlaubt, die elektrische Elektrodenvorrichtung (64) passieren zu lassen, wenn das elastische Material nicht einer axialen Kompression ausgesetzt ist, und bei der weiterhin das elastische Material einer axialen Kompression ausgesetzt wird, wenn wenigstens einer der Arretiererhebungen (104) in der ersten Vertiefung (109, 110) empfangen wird, wobei die axiale Kompression das elastische Material zwingt, sich in den rohrförmigen Kanal (72) hineinzubauschen und damit den Innendurchmesser des ringförmigen elastischen Abschnittes (103) ausreichend zu verringern, um die elektrische Elektrodenvorrichtung (64) fest einzuspannen und dadurch eine axiale Bewegung derselben zu verhindern.

9. Implantierbare medizinische Vorrichtung nach Anspruch 7, bei der der Durchmesser der ersten Vertiefung (138) größer ist als der Durchmesser der zweiten Vertiefung (140) und bei der der Sperring eine radial kompressible Hülse (130) aufweist, die einen ersten Innendurchmesser hat, der es erlaubt, daß die elektrische Elektrodenvorrichtung (64) hindurchgleitet, wenn die Schulter der Hülse (130) innerhalb der ersten Vertiefung (138) empfangen wird, und bei der die Hülse (130) einen zweiten Innendurchmesser hat, der sperrend die elektrische Elektrodenvorrichtung (64) einspannt, wenn die Schulter der Hülse in der zweiten Vertiefung (140) empfangen wird (Fig.7).

10. Implantierbare medizinische Vorrichtung nach Anspruch 1, bei der die Sperrmittel Verbindungsmittel auf der Innenseite des rohrförmigen Kanals (72'') aufweisen, die zu passenden Verbindungsmitteln (144) auf der elektrischen Elektrodenvorrichtung (62') passen, wann immer ein proximales Ende der elektrischen Elektrodenvorrichtung (62') in das offene Ende des rohrförmigen Kanals (72'') eingeführt wird (Fig.8).

11. Implantierbare medizinische Vorrichtung nach Anspruch 10, bei der die Verbindungsmittel des rohrförmigen Kanals (72'') und der elektrischen Elektrodenvorrichtung (62') sperrend miteinander verbunden werden, so daß ein Entfernen der elektrischen Elektrodenvorrichtung (62') aus dem rohrförmigen Kanal (72'') verhindert wird, wann immer die elektrische Elektrodenvorrichtung (62') relativ zu dem rohrförmigen Kanal (72'') einen Bruchteil einer Umdrehung gedreht wird (Fig.8).

## Revendications

1. Dispositif médical implantable comprenant:
un boîtier (61) scellé hermétiquement;
un circuit électrique (12) à l'intérieur du boîtier;
un connecteur dans le boîtier, comprenant:
une structure (74) de barillet tubulaire ayant une extrémité ouverte (78) et une extrémité fermée (76), et un axe de barillet passant par l'extrémité ouverte (78) et l'extrémité fermée (76), l'extrémité ouverte (78) étant fixée à une surface exposée du boîtier (61), l'extrémité fermée (76) étant intérieure au boîtier (61), la structure (74) de barillet tubulaire créant un canal tubulaire (72) qui fait saillie à l'intérieur du boîtier scellé (61), mais ne rompt pas l'étanchéité du boîtier (61), l'intérieur du canal tubulaire (72) s'ouvrant vers l'extérieur du boîtier scellé (61) à travers l'extrémité ouverte (78),
un moyen pour établir un contact électrique entre le canal tubulaire (72) et le circuit électrique (12), et
un moyen de verrouillage (70) pour verrouiller de façon amovible et serrer un conducteur électrique (64) inséré axialement dans l'extrémité ouverte (78) du canal tubulaire (72),
le moyen de verrouillage pouvant être actionné avec une rotation relative inférieure à 360° entre le conducteur et la structure de barillet tubulaire, caractérisé en ce que:
le canal tubulaire (72) comprend en outre une pluralité de parties (80, 84) cylindriques conductrices coaxiales à l'axe du barillet, la dimension du diamètre de parties (80, 84) cylindriques successives décroissant progressivement de l'extrémité ouverte (78) jusqu'à l'extrémité fermée (76);
le contact électrique étant établi entre des parties sélectionnées parmi les parties cylindriques (80, 84) et le circuit électrique (12); et le conducteur électrique (64) étant en contact électrique avec les parties cylindriques (80, 84) du canal tubulaire (72) qui sont sélectionnées (fig. 4, 6);
dans lequel des parties successives de la pluralité de parties cylindriques (80, 84) conductrices sont séparées par une partie cylindrique (82, 86) non conductrice isolante;
et dans lequel des parties cylindriques (80, 84) conductrices successives de la structure de barillet (74) sont à distance les unes des autres et se chevauchent à leurs extrémités adjacentes respectives, en définissant ainsi entre elles une région de chevauchement, les parties (82, 86) cylindriques non conductrices isolantes étant disposées dans toute la région de chevauchement entre des parties cylindriques (80, 84) conductrices successives, les extrémités des parties cylindriques (80, 84) conductrices étant ainsi maintenues à distance les unes des autres sans se toucher et électriquement isolées les unes des autres par les parties cylindriques (82, 86) non conductrices isolantes.

2. Dispositif médical implantable selon la revendication 1, dans lequel le boîtier (61) scellé hermétiquement ne doit pas présenter un taux de fuite supérieur à 2 x 10⁻⁹ Nm/s (2 x 10E-8 atm-c³/s).

3. Dispositif médical implantable selon la revendication 1, dans lequel le boîtier (61) scellé hermétiquement comprend un stimulateur cardiaque (60), et le conducteur électrique (64) comprend un conducteur (64) pour stimulateur cardiaque adapté à connecter le stimulateur cardiaque (60) à un emplacement souhaité d'un tissu.

4. Dispositif médical implantable selon la revendication 1, dans lequel le moyen de verrouillage (70) peut être actionné sensiblement sans aucune rotation relative entre le conducteur électrique (64) et la structure (74) de barillet tubulaire.

5. Dispositif médical implantable selon la revendication 1, dans lequel le moyen pour établir un contact électrique comprend:
un ressort à enroulement incliné (90, 96) positionné sur l'intérieur des parties cylindriques (80, 84) sélectionnées de façon à établir un contact électrique avec celles-ci en une multiplicité d'emplacements; et
un moyen (94, 100) à fil métallique pour établir un contact électrique avec l'extérieur des parties cylindriques (80, 84) sélectionnées et le circuit électrique (12).

6. Dispositif médical implantable selon l'une quelconque des revendications précédentes, dans lequel
le moyen de verrouillage comprend:
des premier (109, 110) et second (108, 109)
moyens d'engagement à distance les uns des autres sur l'intérieur du canal tubulaire;
une bague de verrouillage (70) ayant une pluralité de bosses de positionnement (104), la bague de verrouillage (70) ayant un diamètre intérieur permettant au conducteur électrique (64) d'être ajusté à travers celle-ci et un diamètre extérieur qui permet à la bague (70) d'être insérée de façon coulissante dans l'extrémité ouverte (78) du canal tubulaire (72), au moins une bosse de positionnement (104) de la bague de verrouillage (70) étant reçue par les premiers (109, 110) ou deuxième (108, 109) moyens d'engagement selon la profondeur à laquelle la bague de verrouillage (70) est insérée dans le canal tubulaire (72);
un moyen de serrage destiné à serrer le conducteur électrique chaque fois qu'au moins une bosse de positionnement (104) est reçue par le premier moyen d'engagement (109, 110), et destiné à libérer le conducteur électrique chaque fois qu'au moins une bosse de positionnement (104) est reçue par le second moyen d'engagement (108, 109).

7. Dispositif médical implantable selon la revendication 6, dans lequel les premier (109, 110) et second (108, 109) moyens d'engagement comprennent des première (109, 110) et seconde (108, 109) gorges circonférentielles sur l'intérieur du canal tubulaire (72) à proximité de son extrémité ouverte (78); et dans lequel au moins l'une des bosses de positionnement (104) de la bague de verrouillage (704) s'encliquette dans l'une ou l'autre des première (109, 110) et seconde (108, 109) gorges selon la profondeur à laquelle la bague de verrouillage (70) est repoussée à l'intérieur du canal tubulaire (72).

8. Dispositif médical implantable selon la revendication 7, dans lequel le canal tubulaire (72) de la structure de barillet (74) comporte en outre un moyen de butée positionné à proximité desdites première (109, 110) et seconde (108, 109) gorges circonférentielles, et dans lequel ledit moyen de serrage est maintenu captif entre le moyen de butée et la bague de verrouillage (70), le moyen de serrage comprenant une section annulaire (103) en matériau élastique, ayant un diamètre intérieur qui permet au conducteur électrique (64) de passer dans celui-ci lorsque le matériau élastique n'est pas soumis à une compression axiale, et dans lequel le matériau élastique est en outre soumis à une compression axiale lorsque ladite au moins une bosse de positionnement (104) est reçue à l'intérieur de la première gorge (109, 110), cette compression axiale provoquant un renflement du matériau élastique vers l'intérieur du canal tubulaire (72), ce qui réduit suffisamment le diamètre intérieur de la section élastique annulaire (103) pour serrer fermement le conducteur électrique (64) afin d'empêcher son mouvement axial.

9. Dispositif médical implantable selon la revendication 7, dans lequel le diamètre de la première gorge (138) est supérieur au diamètre de la seconde gorge (140), et dans lequel la bague de verrouillage comprend un collet (130) compressible radialement, ce collet ayant un premier diamètre intérieur qui permet au conducteur électrique (64) de passer en coulissant dans celui-ci lorsque l'épaulement du collet (130) est reçu à l'intérieur de la première gorge (138), et dans lequel le collet (130) présente un second diamètre intérieur qui serre de façon verrouillable le conducteur électrique (64) lorsque l'épaulement du collet est reçu à l'intérieur de la seconde gorge (140) (fig. 7).

10. Dispositif médical implantable selon la revendication 1, dans lequel le moyen de verrouillage comprend des moyens d'engagement sur l'intérieur du canal tubulaire (72'') qui s'emboîtent dans des moyens d'engagement adaptés (144) sur le conducteur électrique (62') chaque fois qu'une extrémité proximale du conducteur électrique (62') est insérée dans l'extrémité ouverte du canal tubulaire (72'') (fig. 8).

11. Dispositif médical implantable selon la revendication 10, dans lequel les moyens d'engagement du canal tubulaire (72'') et du conducteur électrique (62') entrent en contact de façon verrouillable de manière à empêcher que le conducteur électrique (62') sorte du canal tubulaire (72'') chaque fois que le conducteur électrique (62') est mis en rotation d'une fraction de tour par rapport au canal tubulaire (72'') (fig. 8).
